(19) **Europäisches Patentamt / European Patent Office / Office européen des brevets**

(11) **EP 4 462 440 A1**

(12) **EUROPÄISCHE PATENTANMELDUNG**

(43) Veröffentlichungstag:
**13.11.2024   Patentblatt 2024/46**

(21) Anmeldenummer: 23173133.2

(22) Anmeldetag: **12.05.2023**

(51) Internationale Patentklassifikation (IPC):
***G16C 20/10*** *(2019.01)*

(52) Gemeinsame Patentklassifikation (CPC):
**G16C 20/10**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **OMV Downstream GmbH**
**1020 Wien (AT)**

(72) Erfinder:
• **LECHLEITNER, Andreas**
  **1110 Wien (AT)**
• **LORBACH, Sebastian-Mark**
  **1100 Wien (AT)**

(74) Vertreter: **SONN Patentanwälte GmbH & Co KG**
**Riemergasse 14**
**1010 Wien (AT)**

(54) **BESTIMMUNG VON WERTEN VON REAKTIONSPARAMETERN EINES REAKTIONSMODELLS FÜR EINE PYROLYSE**

(57) Computerimplementiertes Verfahren zur Bestimmung von Werten von Reaktionsparametern eines Reaktionsmodells für eine Pyrolyse eines Kunststoff-Ausgangsmaterials in Kunststoff-Produkte, das Verfahren umfassend die folgenden Schritte:
- Bereitstellen von Versuchsdaten umfassend eine Mehrzahl von Siedepunktverteilungen von Kunststoff-Produkten, wobei jede Siedepunktverteilung einer Pyrolysetemperatur und einer Pyrolysedauer zugeordnet ist, wobei die Siedepunktverteilungen jeweils in eine Anzahl N Lumps $L_1$ bis $L_N$ gebinnt sind, wobei jedem Lump ein Siedepunkt-Temperaturbereich zugeordnet ist;

- Bestimmen jeweils eines Wertes der Reaktionsparameter durch Fitten der Reaktionsparameter des Reaktionsmodells an die Versuchsdaten; wobei das Reaktionsmodell für jeden Lump $L_i$ von $L_1$ bis $L_{N-1}$ eine durch mindestens einen Reaktionsparameter bestimmte Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ aufweist, wobei das Reaktionsmodell weniger als $N*(N-1)/2$ Teilreaktionen aufweist.

Fig. 3

EP 4 462 440 A1

**EP 4 462 440 A1**

**Beschreibung**

Die Erfindung betrifft ein computerimplementiertes Verfahren zur Bestimmung von Werten von Reaktionsparametern eines Reaktionsmodells für eine Pyrolyse eines Kunststoff-Ausgangsmaterials in Kunststoff-Produkte, ein Verfahren zur Durchführung einer Pyrolyse eines Kunststoff-Ausgangsmaterials in Kunststoff-Produkte, ein System zur Datenverarbeitung zur Ausführung des computerimplementierten Verfahrens und ein entsprechendes Computerprogrammprodukt.

Alleine Europa fallen jährlich mehrere Millionen Tonnen an Kunststoffabfällen an, von denen nur ein relativ geringer Anteil für Recycling gesammelt wird. Daher werden große Mengen an Kunststoffabfällen verbrannt, deponiert oder verschifft. Um Recyclingquoten zu erhöhen, kann beispielsweise ein Pfandsystem eingeführt werden, Kunststoffabfälle können mechanisch recycelt werden oder beispielsweise chemisch durch Depolymerisation und erneute Polymerisation wiederaufbereitet werden. Eine große Herausforderung im Zusammenhang mit Kunststoffabfällen ist die Sortierung der verschiedenen Kunststoffarten und die Verunreinigung der Abfälle. Eine verhältnismäßig einfache Möglichkeit, gemischte oder sogar mit anderen Abfällen kontaminierte Kunststoffabfälle zu verarbeiten, bietet die Pyrolyse. Bei pyrolytischen Prozessen werden aus organischen Stoffen bei hohen Temperaturen und in Abwesenheit von Sauerstoff wertvolle Ressourcen erzeugt, die als Ausgangsmaterial für die Wiederverwendung der Kunststoffabfälle verwendet werden können. Im Zuge der Pyrolyse werden mittels thermischer Energie langkettige Kohlenwasserstoffe der Kunststoffabfälle in Moleküle mit geringerem Molekulargewicht gespalten. Die Produkte der Pyrolyse sind flüssig oder gasförmig und können in bereits bestehender Infrastruktur wie beispielsweise in einer herkömmlichen Erdölraffinerie weiterverarbeitet werden.

Die chemischen Prozesse, die während der Pyrolyse stattfinden, sind aufgrund ihrer hohen Komplexität im Detail kaum theoretisch zu beschreiben. Um geeignete Parameter, wie die Pyrolysedauer und die Pyrolysetemperatur, einer geplanten Pyrolyse zu finden, kann die Pyrolyse mittels eines sogenannten Lump-Modells in vereinfachter Form modelliert werden. Die Pyrolyse-Produkte werden dabei typischerweise entsprechend ihres Siedepunkts in sogenannte Lumps klassifiziert. Ein Lump beinhaltet Pyrolyse-Produkte eines bestimmten Siedepunkt-Temperaturbereichs. Die einzelnen Lumps sind im Rahmen bekannter Lump-Modelle typischerweise über monomolekulare, irreversible Reaktionen erster Ordnung miteinander verbunden. Pyrolyse-Produkte eines Lumps mit einem hohen Siedepunkt-Temperaturbereich können im Rahmen der Lump-Modelle in Pyrolyse-Produkte jedes Lumps mit niedrigerem Siedepunkt-Temperaturbereich zerfallen. Der umgekehrte Prozess, d.h. die Polymerisation, ist im Rahmen der Pyrolyse vernachlässigbar und wird im Rahmen von Lump-Modellen typischerweise nicht modelliert. Jede der Reaktionen wird derart modelliert, dass eine Rate oder eine Geschwindigkeit der jeweiligen Reaktion eines Lumps in einen anderen Lump angegeben werden kann. Zur Bestimmung der Parameter der einzelnen Reaktionen werden typischerweise experimentelle Daten eines Versuchsreaktors verwendet.

Beispielsweise zeigt Lechleitner, A.E.; Schubert, T.; Hofer, W.; Lehner, M. Lumped Kinetic Modeling of Polypropylene and Polyethylene Co-Pyrolysis in Tubular Reactors. Processes 2021, 9, 34, https://doi.org/10.3390/pr9010034, ein Lump Modell für die Pyrolyse von Polypropylen und Polyethylen in einem Rohrreaktor ("tubular reactor"). Zur Beschreibung der Pyrolyse wird ein Modell mit vier Lumps verwendet, um die durch die Pyrolyse von Plastik entstehenden Produkte zu klassifizieren. Alle Lumps sind jeweils mittels einer monomolekularen, irreversiblen Reaktion erster Ordnung miteinander verbunden. Zusätzlich ist ein initialer Prozessschritt vorgesehen, in dem das Ausgangsmaterial ("Plastic"), das keinem Siedepunkt-Temperaturbereich zugeordnet wird, in Produkte des Lumps mit dem höchsten Siedepunkt-Temperaturbereich ("Wax 420°C+") umgewandelt wird. Dieser initiale Prozessschritt konnte laut der Publikation nicht evaluiert werden (vgl. Kapitel 2.2.1, letzter Absatz). Die insgesamt sechs Reaktionen $k_i$ werden mittels der Arrhenius-Gleichung modelliert, um die Temperaturabhängigkeit der Reaktionen zu erfassen. Pro Reaktion $k_i$ werden jeweils eine Arrhenius-Konstante $A_i$ und eine Aktivierungsenergie $E_{A,i}$, also insgesamt zwölf Parameter, mittels experimenteller Daten bestimmt.

Ein weiteres Modell für die Pyrolyse von LDPE ("low-density polyethylene") ist bekannt aus Schubert, Teresa, et al. "4-Lump kinetic model of the co-pyrolysis of LDPE and a heavy petroleum fraction." Fuel 262 (2020): 116597. Das Modell weist vier Lumps auf, die wiederum jeweils mittels monomolekularer, irreversibler Reaktionen erster Ordnung miteinander verbunden sind.

Laut Naik, Desavath V., et al. "Kinetic modeling for catalytic cracking of pyrolysis oils with VGO in a FCC unit." Chemical Engineering Science 170 (2017): 790-798 treten bei der Berechnung von kinetischen Parametern typischerweise zwei Probleme auf. Einerseits kommt es zu Problemen bei der Konvergenz bei einer zu großen Anzahl an Parametern. Andererseits kann die Initialisierung der Parameter dazu führen, dass die Optimierung ein lokales Minimum erreicht (womit inkorrekte Parameter berechnet werden würden). Die Publikation zeigt ein Modell mit 5 Lumps für das katalytische Cracken von Pyrolyseölen. Die Parameter des 5-Lump-Modell wurden sequentiell mittels eines 3-Lump und eines 4-Lump-Modells bestimmt, um die Anzahl an simultan zu bestimmenden Parameter gering zu halten.

Es ist Aufgabe der Erfindung, die Nachteile des Stands der Technik zu lindern oder zu beseitigen. Insbesondere ist es Aufgabe der Erfindung, ein computerimplementiertes Verfahren, ein System zur Datenverarbeitung und ein Computerprogrammprodukt zur Bestimmung von Werten von Reaktionsparametern eines Reaktionsmodells für eine Pyrolyse eines Kunststoff-Ausgangsmaterials in Kunststoff-Produkte bereitzustellen, das eine einfache, robuste und effiziente Modellierung der Pyrolyse erlaubt ohne dabei die Genauigkeit der Modellierung signifikant zu verschlechtern.

Gelöst wird die Aufgabe durch ein computerimplementiertes Verfahren zur Bestimmung von Werten von Reaktionsparametern eines Reaktionsmodells für eine Pyrolyse eines Kunststoff-Ausgangsmaterials in Kunststoff-Produkte, das Verfahren umfassend die folgenden Schritte:

- Bereitstellen von Versuchsdaten umfassend eine Mehrzahl von Siedepunktverteilungen von Kunststoff-Produkten, wobei jede Siedepunktverteilung einer Pyrolysetemperatur und einer Pyrolysedauer zugeordnet ist, wobei die Siedepunktverteilungen jeweils in eine Anzahl N Lumps $L_1$ bis $L_N$ gebinnt sind, wobei jedem Lump ein Siedepunkt-Temperaturbereich zugeordnet ist;
- Bestimmen jeweils eines Wertes der Reaktionsparameter durch Fitten der Reaktionsparameter des Reaktionsmodells an die Versuchsdaten;

wobei das Reaktionsmodell für jeden Lump $L_i$ von $L_1$ bis $L_{N-1}$ eine durch mindestens einen Reaktionsparameter bestimmte Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ aufweist, wobei das Reaktionsmodell weniger als N*(N-1)/2 Teilreaktionen aufweist.

Die bisher aus dem Stand der Technik bekannten Reaktionsmodelle, wie etwa beschrieben in der oben genannten Publikation Lechleitner et al. (Processes 2021, 9, 34) umfassen die Teilreaktionen jedes einzelnen Lumps in sämtliche in der Siedepunkt-Temperaturbereich darunter liegenden Lumps. Dies hat zur Folge, dass die Komplexität des Modells mit steigender Anzahl Lumps rasch ansteigt, da N*(N-1)/2 Teilreaktionen berücksichtigt werden müssen. Im Stand der Technik findet sich deswegen auch die Bestrebung, mit möglichst wenigen Lumps auszukommen. So gingen auch Lechleitner et al. Ursprünglich von einem 6-Lump-Modell aus, dass in der Folge zu einem 4-Lump-Modell vereinfacht wurde (vgl. Lechleitner et al., Kapitel 2.2.1).

Im Zuge der vorliegenden Erfindung hat sich nun überraschenderweise herausgestellt, dass einige Teilreaktionen vernachlässigt werden können, ohne die Genauigkeit des Reaktionsmodells signifikant zu verschlechtern. Im Vergleich zum Stand der Technik ist die Anzahl der Teilreaktionen daher auf weniger als N*(N-1)/2 reduziert. Durch die Reduktion der Teilreaktionen kann die Konvergenz des Fittens verbessert werden und generell die Modellkomplexität bei gleicher Anzahl Lumps verringert werden - bzw. bei gleicher Modellkomplexität die Anzahl der berücksichtigten Lumps erhöht werden. Entscheidend dabei ist die Erkenntnis, welche Teilreaktionen vernachlässigt werden können und welche keinesfalls vernachlässigt werden können, ohne die Genauigkeit des Reaktionsmodells signifikant zu verschlechtern. Die für die akkurate Beschreibung der Pyrolyse entscheidendsten Teilreaktionen sind solche zwischen in Bezug auf den Siedepunkt-Temperaturbereich benachbarten Lumps. Es hat sich herausgestellt, dass Teilreaktionen zwischen Lumps, die in Bezug auf den Siedepunkt-Temperaturbereich einen Abstand aufweisen, in der Pyrolyse weniger häufig vorkommen und für die Genauigkeit des Reaktionsmodells weniger kritisch sind. Im Umkehrschluss sind die Reaktionsparameter dieser weniger entscheidenden Teilreaktionen schwieriger zu bestimmen, da die Versuchsdaten diese Teilreaktionen nur untergeordnet beinhalten und daher die Bestimmung der Werte der betreffenden Reaktionsparameter größeren experimentellen wie auch statistischen Unsicherheiten unterliegen. Die Modellierung solcher wenig häufigen Teilreaktionen kann insgesamt die Konvergenz des Fits negativ beeinflussen. Daher hat es sich als in Summe entscheidend herausgestellt, dass das Reaktionsmodell für jeden Lump $L_i$ von $L_1$ bis $L_{N-1}$ eine Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ aufweist, da diese Reaktionen die zur Beschreibung der Pyrolyse am kritischsten sind.

Im Rahmen einer Pyrolyse eines Kunststoff-Ausgangsmaterials in Kunststoff-Produkte wird dem Kunststoff-Ausgangsmaterial thermische Energie zugeführt, wodurch langkettige Moleküle des Kunststoff-Ausgangsmaterials gespalten werden und Kunststoff-Produkte gebildet werden. Als Kunststoff-Ausgangsmaterial kann beispielsweise jegliche Art von Kunststoff-Abfall verwendet werden. Beispielsweise kann das Kunststoff-Ausgangsmaterial Polypropylen oder Polyethylen aufweisen. Die Kunststoff-Produkte können beispielsweise (vergleichsweise kürzerkettige und/oder leichtere) Kohlenwasserstoffe aufweisen, wie beispielsweise Schweröle, Spindelöle, Gasöle, Kerosin, Naphtha, Flüssiggas (LPG) oder bei Normalbedingungen gasförmige Kohlenwasserstoffe.

Die Pyrolyse kann in einem Pyrolyse-Reaktor durchgeführt werden. Beispielweise zeigt Lechleitner, A.E.; Schubert, T.; Hofer, W.; Lehner, M. Lumped Kinetic Modeling of Polypropylene and Polyethylene Co-Pyrolysis in Tubular Reactors. Processes 2021, 9, 34, https://doi.org/10.3390/pr9010034 einen Pyrolyse-Reaktor, der als Rohrreaktor ("tubular reactor") ausgeführt ist. Entscheidende Parameter der Pyrolyse sind die Pyrolyse-Temperatur, d.h. die Temperatur auf die das Kunststoff-Ausgangsmaterial erhitzt wird, sowie die Pyrolysedauer, die angibt, wie lange die Pyrolyse-Temperatur gehalten wird. Im Allgemeinen gilt, dass sowohl eine höhere Pyrolyse-Temperatur als auch eine höhere Pyrolysedauer zur verstärkten Umwandlung in kürzerkettige Kunststoff-Produkte führt.

Ein Schritt des Verfahrens betrifft das Bereitstellen von Versuchsdaten umfassend eine Mehrzahl von Siedepunktverteilungen von Kunststoff-Produkten. Die Versuchsdaten können beispielsweise mittels einer Mehrzahl an Pyrolyseläufen gewonnen werden, im Rahmen derer jeweils mit dem gleichen Kunststoff-Ausgangsmaterial in demselben Pyrolyse-Reaktor eine Pyrolyse bei einer Pyrolysetemperatur und einer Pyrolysedauer durchgeführt wird. Jeder Pyrolyselauf führt zu Kunststoff-Produkten, die entsprechend ihres Siedepunkts klassifiziert werden können. Jeder experimentell ermittelten Siedepunktverteilung ist eine Pyrolysetemperatur und eine Pyrolysedauer zugeordnet. Vorzugsweise weisen die Versuchsdaten Siedepunktverteilungen von einer Mehrzahl unterschiedlicher Pyrolysetemperaturen und/oder einer Mehrzahl unterschiedlicher Pyrolysedauern auf. Die Siedepunktverteilungen sind jeweils in eine Anzahl N Lumps $L_1$ bis $L_N$ gebinnt, wobei jedem Lump ein Siedepunkt-Temperaturbereich zugeordnet ist. Eine gemessene Siedepunktverteilung kann beispielsweise ursprünglich kontinuierlich als Funktion der Siedepunkt-Temperatur vorliegen, in die Lumps gebinnt werden und folglich in Form eines Histogramms vorliegen. Beispielsweise können neun Lumps $L_1$ bis $L_9$ vorgesehen sein, in die die einzelnen Siedepunktverteilungen jeweils gebinnt sind. Der erste Lump $L_1$ kann beispielsweise das Kunststoff-Ausgangsmaterial und einen Siedepunkt-Temperaturbereich über 600°C aufweisen. Der zweite Lump $L_2$ kann beispielsweise einen Siedepunkt-Temperaturbereich zwischen 450°C und 600°C aufweisen und kann als Sumpfprodukt oder Schwerprodukt bezeichnet werden. Der dritte Lump L3 kann Schweröl aufweisen und kann einen Siedepunkt-Temperaturbereich zwischen 400°C und weniger als 450°C aufweisen. Der vierte Lump L4 kann Spindelöl aufweisen und kann einen Siedepunkt-Temperaturbereich zwischen 350°C und weniger als 400°C aufweisen. Der fünfte Lump L5 kann Gasöl aufweisen und kann einen Siedepunkt-Temperaturbereich zwischen 225°C und weniger als 350°C aufweisen. Der sechse Lump L6 kann beispielsweise Kerosin aufweisen und einen Siedepunkt-Temperaturbereich zwischen 165°C und weniger als 225°C aufweisen. Der siebte Lump L7 kann beispielsweise Naphtha aufweisen und einen Siedepunkt-Temperaturbereich zwischen 20°C und weniger als 165°C aufweisen. Der achte Lump L8 kann beispielsweise LPG ("Liquified Petroleum Gas") aufweisen und einen Siedepunkt-Temperaturbereich zwischen -120°C und weniger als 20°C aufweisen. Der achte Lump L8 kann in diesem Beispiel überwiegend Kohlenwasserstoffe mit einer Anzahl an Kohlenwasserstoffatomen zwischen zwei und vier aufweisen (beispielsweise Ethen, Ethan, Propan, Propen, Butan und/oder Buten). Der neunte Lump L9 kann beispielsweise bei Normalbedingungen gasförmige Kohlenwasserstoffe aufweisen mit einem Siedepunkt kleiner als -120°C. Der neunte Lump L9 kann in diesem Fall überwiegend Methan aufweisen. Alternativ können mehr oder weniger Lumps vorgesehen sein. Beispielsweise können zwei oder mehrere der oben beispielhaft angeführten Lumps zusammengefasst sein. Beispielsweise können andere Lumps mir anderen Siedepunt-Temperaturbereichen vorgesehen sein.

Im nächsten Schritt wird jeweils ein Wert der Reaktionsparameter durch Fitten der Reaktionsparameter des Reaktionsmodells an die Versuchsdaten bestimmt. Das Reaktionsmodell beschreibt die Umwandlung der Kunststoff-Produkte und des Kunststoff-Ausgangsmaterials der jeweiligen Lumps in Kunststoff-Produkte anderer Lumps. Die Lumps sind gemäß dem Modell vorzugsweise mit monomolekularen, irreversiblen Teilreaktionen erster Ordnung verbunden. Beispielsweise ist Lump L1 mit Lump L2 mit einer Teilreaktion $r_{12}$ verbunden, wobei folgende Relation gilt: $r_{12} = k_{12} \cdot X_{Lump1}$. $k_{12}$ ist eine Reaktionsrate zwischen Lump L1 und Lump L2. $X_{Lump1}$ ist ein Masseanteil an Kunststoff-Produkten in Lump L1 (relativ zur Gesamtmasse aller Lumps). Die einzelnen Teilreaktionen können mittels der Arrhenius Gleichung beschrieben werden, um die Abhängigkeit der Pyrolysetemperatur T zu berücksichtigen:

$$k_{12} = k_{12}^* \times \exp\left(-\frac{E_{A12}}{R\,T}\right)$$

$k_{12}^*$ ist eine Arrhenius-Konstante (auch als prä-exponentieller Faktor oder Frequenzfaktor bekannt). $E_{A12}$ beschreibt die Aktivierungsenergie der jeweiligen Umwandlung, in diesem Fall die Aktivierungsenergie für eine Umwandlung von Kunststoff Produkten von Lump L1 in Lump L2. R ist die universelle Gaskonstante und T ist die (absolute) Pyrolysetemperatur. Für den Fall, dass jede Teilreaktion mittels einer Arrhenius-Gleichung modelliert wird, wird folglich jede Teilreaktion mittels zweier Reaktionsparameter beschrieben. Die Reaktionsparameter pro Teilreaktion von einem Lump Li zu einem Lump Lj sind in diesem Fall $k_{ij}^*$ und $E_{Aij}$.

Das Reaktionsmodell weist für jeden Lump $L_i$ von $L_1$ bis $L_{N-1}$ eine durch mindestens einen Reaktionsparameter bestimmte Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ auf. i ist ein Index, der ganzzahlige positive Werte zwischen eins und der Anzahl an Lumps N annehmen kann. Das Reaktionsmodell bezieht sich auf die Pyrolyse in einem Pyrolyse-Reaktor. Die Versuchsdaten sind experimentelle Daten, die mittels des Pyrolyse-Reaktors gewonnen wurden. Das Reaktionsmodell ist eine Funktion der Pyrolysetemperatur und der Pyrolysedauer.

Ein Pyrolysemodell mit drei Lumps L1, L2 und L3, in dem eine Teilreaktion von Lump L1 zu Lump L2 und eine Teilreaktion von Lump L2 zu Lump L3 berücksichtigt wird, kann beispielsweise die folgende Form aufweisen.

$$k_{12} = k_{12}^{*} \times exp(-\frac{E_{A12}}{R\,T})$$

$$k_{23} = k_{23}^{*} \times exp(-\frac{E_{A23}}{R\,T})$$

$$\frac{dX_1}{dt} = -k_{12} \times X_1$$

$$\frac{dX_2}{dt} = -k_{23} \times X_2 + k_{12} \times X_1$$

$$\frac{dX_3}{dt} = k_{23} \times X_2$$

$X_1$, $X_2$ und $X_3$ bezeichnen den jeweiligen Masseanteil in Lump L1, Lump L2 bzw. Lump L3. t bezeichnet die Pyrolysedauer. Die Reaktionsraten $k_{12}$ und $k_{23}$ können mittels der Arrhenius Gleichung beschrieben werden, in die auch die Pyrolysetemperatur T eingeht. $X_1$, $X_2$ und $X_3$ sind in diesem Fall Funktionen der Pyrolysedauer t und der Pyrolysetemperatur T, daher gilt beispielsweise $X_1 = X_1(t,T)$. In diesem vereinfachten Fall weist das Pyrolysemodell insgesamt vier Reaktionsparameter auf: $k_{12}^{*}$ und $E_{A12}$, sowie $k_{23}^{*}$ und $E_{A23}$, deren Werte mittels des erfindungsgemäßen Verfahrens bestimmt werden können.

Die Versuchsdaten stellen experimentelle Daten von Pyrolyseläufen zu jeweils einem einzelnen Wertepaar aus Pyrolysetemperatur und Pyrolysedauer dar. Mittels des Reaktionsmodells kann zwischen den experimentellen Daten zu diesen Wertepaaren interpoliert werden oder darüber hinaus extrapoliert werden. Mittels des Reaktionsmodells kann die Siedepunktverteilung von Kunststoff-Produkten einer Pyrolyse mit einer beliebigen Pyrolysetemperatur und einer beliebigen Pyrolysedauer abgeschätzt werden. Typischerweise liegt die Pyrolysetemperatur zwischen 300°C und 650°C. Die Pyrolysedauer liegt typischerweise zwischen wenigen Minuten und bis zu einigen Stunden. Beispielsweise kann die Pyrolysedauer einen Wert zwischen 3 Minuten und 100 Minuten aufweisen.

Die Werte der Reaktionsparameter werden durch Fitten der Reaktionsparameter des Reaktionsmodells an die Versuchsdaten bestimmt. Fitten bedeutet in diesem Fall, dass die Werte der Reaktionsparameter so gewählt werden, dass das Reaktionsmodell die Versuchsdaten möglichst exakt wiedergibt. Beispielsweise kann eine Summe von Abstandsquadraten des Reaktionsmodells zu den Versuchsdaten den kleinstmöglichen Wert annehmen (auch als "least squares" Verfahren oder Methode der kleinsten Quadrate bekannt). Fitten kann beispielsweise Minimieren eines Abstands von den Versuchsdaten zum Reaktionsmodell beinhalten. Der Abstand kann beispielsweise mittels einer Zielfunktion berechnet werden. Beispielsweise können im Rahmen des Fittens die Reaktionsparameter variiert werden, sodass ein minimaler Abstand der Versuchsdaten zum Reaktionsmodell aufgefunden wird. Die so bestimmten Werte der Reaktionsparameter führen zu dem minimalen Abstand des Reaktionsmodells zu den Versuchsdaten.

Im Stand der Technik sind Reaktionsmodelle mit N*(N-1)/2 Teilreaktionen bekannt, d.h. es werden Teilreaktionen zwischen allen Lumps berücksichtigt. Im Vergleich zum Stand der Technik ist die Anzahl der Teilreaktionen auf weniger als N*(N-1)/2 reduziert. Durch die Reduktion des Teilreaktionen kann die Konvergenz des Fittens verbessert werden und generell die Modellkomplexität verringert werden. Entscheidend dabei ist, welche Teilreaktionen vernachlässigt werden können und welche keinesfalls vernachlässigt werden können, ohne die Genauigkeit des Reaktionsmodells signifikant zu verschlechtern. Wie oben näher beschrieben, ist es in Summe entscheidend, dass das Reaktionsmodell für jeden Lump $L_i$ von $L_1$ bis $L_{N-1}$ eine Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ aufweist, da diese Reaktionen die zur Beschreibung der Pyrolyse kritischsten sind.

Beispielsweise kann das Reaktionsmodell keine Teilreaktion $r_{1N}$ für eine Reaktion zwischen dem ersten Lump $L_1$ und dem letzten Lump $L_N$ aufweisen. Diese Teilreaktion weist den größten Abstand im Siedepunkt-Temperaturbereich der beiden beteiligten Lumps auf und ist demnach besonders wenig relevant für die Beschreibung der Pyrolyse. Umgekehrt können speziell die Reaktionsparameter dieser Teilreaktion $r_{1N}$ im Rahmen des Fittens nicht einfach zu initialisieren sein und auch die Konvergenz des Fits negativ beeinflussen.

Im Allgemeinen kann das Reaktionsmodell N Lumps aufweisen und weniger als N*(N-1)/M Teilreaktionen aufweisen, wobei M eine natürliche Zahl größer zwei ist, die kleiner als die Anzahl an Lumps N ist (d.h. N > M). Beispielsweise kann das Reaktionsmodel eine Anzahl N von mindestens 4 Lumps und weniger als N*(N-1)/3 Teilreaktionen aufweisen.

Die Anzahl N an Lumps beträgt vorzugsweise zumindest drei, mehr bevorzugt zumindest vier, mehr bevorzugt zumindest fünf, mehr bevorzugt zumindest sechs, mehr bevorzugt zumindest sieben, mehr bevorzugt zumindest acht, mehr bevorzugt zumindest neun, mehr bevorzugt zumindest zehn, mehr bevorzugt zumindest elf, mehr bevorzugt zumindest zwölf. Die Anzahl N an Lumps kann beispielsweise zumindest sieben betragen. Beispielsweise kann die Anzahl an Lumps zumindest zwanzig betragen. Aufgrund des Reaktionsmodells mit einem im Vergleich zum Stand der Technik kleineren Verhältnis von Teilreaktionen bzw. Reaktionsparametern zur Anzahl an Lumps, ist es möglich eine verhältnismäßig große Anzahl an Lumps zu verwenden, ohne die Konvergenz des Fits zu beeinträchtigen. Dies ermöglicht es, die Granularität der Vorhersage zu erhöhen, ohne die Vorhersagequalität signifikant zu verschlechtern.

Beispielsweise kann das Reaktionsmodell genau (N-1) Teilreaktionen aufweisen. In diesem Fall berücksichtigt das Reaktionsmodell ausschließlich Teilreaktionen zwischen in Bezug auf den Siedepunkt-Temperaturbereich benachbarten Lumps. Das Reaktionsmodell kann in diesem Fall als sequentielles Reaktionsmodell bezeichnet werden und weist ausschließlich sequentielle Teilreaktionen auf. In diesem Fall bildet das Reaktionsmodell einen einzigen Reaktionspfad vom ersten Lump L1 zum letzten Lump LN ab. Beispielsweise kann eine Umwandlung von einem ersten Lump L1 in einen dritten Lump L3 nur über eine Umwandlung von Lump L1 in einen zweiten Lump L2 und einer weiteren Umwandlung von Lump L2 in Lump L3 erfolgen, wobei Lump 2 in Bezug auf den Siedepunkt-Temperaturbereich zwischen Lump L1 und Lump L3 liegt und sowohl an Lump L1 als auch an Lump L3 angrenzt. Alternative Pfade, beispielsweise eine direkte Umwandlung von Lump L1 in Lump L3 (die in Bezug auf den Siedepunkt-Temperaturbereich nicht aneinander angrenzen) sind im Rahmen des sequentiellen Reaktionsmodells nicht vorgesehen und demnach im Rahmen des Reaktionsmodells nicht möglich. Die sequentiellen Teilreaktionen sind in Bezug auf eine akkurate Beschreibung der Pyrolyse die entscheidendsten Teilreaktionen zwischen den Lumps. Durch die Einschränkung des Reaktionsmodells auf die (N-1) sequentiellen Teilreaktionen können Konvergenzprobleme weitestgehend vermieden werden, weiters hängt der Fit und damit die Werte der Reaktionsparameter nicht von initialen Startwerten der Reaktionsparameter für das Fitten ab. Das Reaktionsmodell bildet die Pyrolyse trotz der Vereinfachung im Vergleich zu bekannten und komplexeren Reaktionsmodellen akkurat ab. Zudem ermöglicht die Einschränkung des Reaktionsmodells auf die (N-1) sequentiellen Teilreaktionen, eine deutlich größere Anzahl an Lumps zu berücksichtigen, ohne insgesamt die Modellkomplexität zu erhöhen.

Die Erfindung betrifft weiters ein Verfahren zur Bestimmung von Werten Reaktionsparametern eines Reaktionsmodells für eine Pyrolyse eines Kunststoff-Ausgangsmaterials in Kunststoff-Produkte mit einem erfindungsgemäßen computerimplementierten Verfahren zur Bestimmung von Werten von Reaktionsparametern eines Reaktionsmodells für eine Pyrolyse eines Kunststoff-Ausgangsmaterials in Kunststoff-Produkte, wobei das Bereitstellen von Versuchsdaten folgende Schritte umfasst:

- Durchführen einer Mehrzahl von Pyroläufen mit unterschiedlicher Pyrolysetemperatur und/oder Pyrolysedauer in einem Pyrolyse-Reaktor;
- Messung einer Siedepunktverteilung von Kunststoff-Produkten pro Pyrolyselauf, wobei jede Siedepunktverteilung einer Pyrolysetemperatur und einer Pyrolysedauer zugeordnet ist;
- Binning der einzelnen Siedepunktsverteilungen in die N Lumps $L_1$ bis $L_N$, wobei jedem Lump ein Siedepunkt-Temperaturbereich zugeordnet ist, um Versuchsdaten zu erhalten.

Die Versuchsdaten wurden mittels einer Mehrzahl an Pyroläufen gewonnen, bei denen jeweils mit dem gleichen Kunststoff-Ausgangsmaterial in demselben Pyrolyse-Reaktor eine Pyrolyse bei einer Pyrolysetemperatur und einer Pyrolysedauer durchgeführt wurde. Jeder Pyrolyselauf führt zu Kunststoff-Produkten, die entsprechend ihres Siedepunkts klassifiziert werden können.

Zu jedem Pyrolyselauf wird die Siedepunktverteilung der Kunststoff-Produkte gemessen und so die Kunststoff-Produkte entsprechend ihres Siedepunkts klassifiziert. Lechleitner, A.E.; Schubert, T.; Hofer, W.; Lehner, M. Lumped Kinetic Modeling of Polypropylene and Polyethylene Co-Pyrolysis in Tubular Reactors. Processes 2021, 9, 34. https://doi.org/10.3390/pr9010034 zeigt beispielsweise einen Versuchsreaktor ("pilot plant") der im Anschluss an den Pyrolyse-Reaktor (d.h. downstream des Pyrolyse-Reaktors) eine Flash Vessel (kann auch als Verdampfer bezeichnet werden) aufweist. Mit Hilfe der Flash-Vessel können Kunststoff-Produkte, die gasförmig vorliegen, abgetrennt werden. Dazu kann eine Temperatur und ein Druck in der Flash-Vessel eingestellt werden, um die Trennung (den sogenannten Trennschnitt) zu beeinflussen. Kunststoff-Produkte, die unter den jeweiligen Bedingungen (d.h. Druck und Temperatur) in der Flash Vessel gasförmig sind, können den Verdampfer verlassen und können zu einer oder mehreren Kühlfallen geleitet werden. Die so entnommenen Kunststoff-Produkte können gewogen, gekühlt und beispielsweise mit einem Sumpfprodukt der Flash-Vessel zu einem flüssigen Endprodukt gemischt werden, das folglich analysiert werden kann. Kunststoff-Produkte, die unterhalb von 0°C gasförmig sind, können beispielsweise mit einem Gasballon gesammelt und danach weiter analysiert werden.

Die Analyse der gasförmigen Kunststoff-Produkte kann beispielsweise mittels Gaschromatographie nach DIN 51666:2007-01 erfolgen. Dadurch kann beispielsweise der Heizwert, das spezifische Gewicht und/oder eine detaillierte molekulare Zusammensetzung der Kunststoff-Produkte ermittelt werden. Die Siedepunktverteilung der gesammelten flüssigen Kunststoff-Produkte und eines etwaigen Trägermediums kann beispielsweise nach ASTM D7169-20e1 mittels simulierter Destillation analysiert werden. Die Siedepunktverteilung ist entsprechend der Masse gewichtet.

Jeder experimentell ermittelten Siedepunktverteilung ist eine Pyrolysetemperatur und eine Pyrolysedauer des jeweiligen Pyrolyselaufs zugeordnet.

Die Siedepunktverteilungen werden jeweils in eine Anzahl N Lumps $L_1$ bis $L_N$ gebinnt, wobei jedem Lump ein Siedepunkt-Temperaturbereich zugeordnet ist, um so die Versuchsdaten zu erhalten. Binnen bedeutet eine Einteilung oder Unterteilung der Siedepunktverteilung in Klassen, wobei die Klassen in diesem Fall die Lumps sind.

Weiters betrifft die Erfindung ein Verfahren zur Durchführung einer Pyrolyse eines Kunststoff-Ausgangsmaterials in Kunststoff-Produkte umfassend die folgenden Schritte:

- Bestimmung von Werten von Reaktionsparametern eines Reaktionsmodells der Pyrolyse durch das erfindungsgemäße computerimplementierte Verfahren;
- Vorgeben einer vorgesehenen Siedepunktverteilung;
- Festlegen einer Pyrolysetemperatur und einer Pyrolysedauer durch Minimieren eines Abstands einer mittels des Reaktionsmodells und der zuvor bestimmten Werte der Reaktionsparameter berechneten Siedepunktverteilung zu der vorgesehenen Siedepunktverteilung; und
- Durchführen der Pyrolyse mit der festgelegten Pyrolysetemperatur und der festgelegten Pyrolysedauer.

Das Pyrolysemodell ist eine Funktion der Pyrolysedauer und der Pyrolysetemperatur. Mittels des Pyrolysemodells mit den zuvor bestimmten Reaktionsparametern der Teilreaktionen kann zu jedem Wertepaar aus Pyrolysedauer und der Pyrolysetemperatur eine (theoretische) Siedepunktverteilung der Kunststoff-Produkte berechnet werden. Die berechnete Siedepunkt-Verteilung weist dieselben Lumps auf, die auch das Pyrolysemodell vorsieht.

In einem folgenden Schritt wird eine vorgesehene Siedepunktverteilung vorgegeben. Die vorgegebene Siedepunktverteilung kann kontinuierlich oder als Histogramm vorliegen. Die vorgegeben Siedepunktverteilung kann beispielsweise dieselben Lumps aufweisen, wie das Reaktionsmodell. Die vorgegebenen Siedepunkt-Verteilung gibt an, welche Siedepunktverteilung die Kunststoff Produkte nach einer Pyrolyse eines Kunststoff-Ausgangsmaterials im Idealfall aufweisen soll. Die vorgesehene Siedepunktverteilung kann angeben, in welche Lumps die Kunststoff-Produkte durch die Pyrolyse bevorzugt umgewandelt werden sollen.

Im nächsten Schritt wird die Pyrolysetemperatur und die Pyrolysedauer festgelegt, indem ein Abstand einer mittels des Reaktionsmodells und der zuvor bestimmten Werte der Reaktionsparameter berechneten Siedepunktverteilung zu der vorgesehenen Siedepunktverteilung minimiert wird. Beispielsweise können die vorgegebene Siedepunktverteilung und die berechnete Siedepunktverteilung jeweils als Histogramm vorliegen und dieselben Lumps aufweisen. Der Abstand kann in diesem Beispiel eine Summe aus den Differenzen jeweils eines vorgegebenen Häufungswertes eines Lumps der vorgegebenen Siedepunkt-Verteilung zu einem entsprechenden berechneten Häufungswertes eines Lumps der berechneten Siedepunktverteilung sein. Der Abstand kann eine Gewichtung aufweisen, beispielsweise kann ein Lump stärker gewichtet sein als die übrigen Lumps, sodass das Modell den stärker gewichteten Lump besonders genau abbildet. Die Pyrolysetemperatur und die Pyrolysedauer sind jeweils Variablen des Reaktionsmodells (und auch der Pyrolyse). Die festgelegte Pyrolysetemperatur und die festgelegte Pyrolysedauer sind diejenige Werte dieser Variablen, die zum minimalen Abstand der vorgegebenen Siedepunkt-Verteilung zu der berechneten Siedepunkt-Verteilung führen.

Im letzten Schritt wird die Pyrolyse mit der festgelegten Pyrolysetemperatur und der festgelegten Pyrolysedauer durchgeführt. Dazu wird der Pyrolyse-Reaktor so betrieben, dass die festgelegte Pyrolysetemperatur und die festgelegte Pyrolysedauer erreicht und eingehalten wird. Beispielsweise kann das gleiche Kunststoff-Ausgangsmaterial verwendet werden, wie bei den zuvor zu Bestimmung der Reaktionsparameter der Teilreaktionen durchgeführten Pyrolyseläufen verwendet wurde. Beispielsweise kann der gleiche Pyrolyse-Reaktor für die Pyrolyse verwendet werden, wie bei den zuvor zu Bestimmung der Reaktionsparameter durchgeführten Pyrolyseläufen verwendet wurde.

Ein weiteres Verfahren zur Durchführung einer Pyrolyse eines Kunststoff-Ausgangsmaterials in Kunststoff-Produkte umfasst die folgenden Schritte:

- Bestimmung von Werten von Reaktionsparametern eines Reaktionsmodells der Pyrolyse durch das erfindungsgemäße computerimplementierte Verfahren;

- Berechnen einer Mehrzahl an Siedepunktverteilungen mittels des Reaktionsmodells und der zuvor bestimmten Werte der Reaktionsparameter, wobei jeder berechneten Siedepunktverteilung eine Pyrolysetemperatur und eine Pyrolysedauer zugeordnet ist;
- Auswählen einer der berechneten Siedepunktverteilungen, um die Pyrolysetemperatur und die Pyrolysedauer festzulegen; und
- Durchführen der Pyrolyse mit der festgelegten Pyrolysetemperatur und der festgelegten Pyrolysedauer.

Die Auswahl der berechneten Siedepunktverteilung kann beispielsweise auf Basis vordefinierten Kriterien erfolgen. Beispielsweise kann eine Siedepunktverteilung ausgewählt werden, die ein Maximum in einem vordefinierten Lump aufweist. Die Pyrolyse mit der festgelegte Pyrolysetemperatur und die festgelegte Pyrolysedauer kann zu einer Siedepunktverteilung der Kunststoff-Produkte führen, die im Wesentlichen der ausgewählten Siedepunktverteilung entspricht.

Die Erfindung betrifft weiters ein System zur Datenverarbeitung aufweisend Mittel zur Ausführung der Schritte des erfindungsgemäßen computerimplementierten Verfahrens. Beispielsweise kann das System zu Datenverarbeitung ein Computer, wie beispielsweise ein Laptop, sein. Beispielsweise kann das System zu Datenverarbeitung einen Prozessor und eine Festplatte aufweisen.

Die Mittel des Systems zur Datenverarbeitung können weiters zur Ausführung des folgenden Schritts eingerichtet sein:

- Berechnen einer Pyrolysetemperatur und einer Pyrolysedauer mittels des Reaktionsmodells und der zuvor bestimmten Werte der Reaktionsparameter, wobei die Pyrolysetemperatur und die Pyrolysedauer derart festgelegt ist, dass ein Abstand einer mittels des Reaktionsmodells berechneten Siedepunktverteilung zu einer vorgesehenen Siedepunktverteilung minimiert ist.

Die Erfindung betrifft weiters ein Computerprogrammprodukt, umfassend Befehle, die bei der Ausführung des Programms durch einen Computer diesen veranlassen, die Schritte des erfindungsgemäßen computerimplementierten Verfahrens.

Die vorliegende Erfindung wird anhand von in den Figuren dargestellten Ausführungsbeispielen weiter erläutert, auf die sie jedoch nicht beschränkt sein soll.

Fig. 1 zeigt schematisch den Aufbau eines Pyrolyse-Reaktors

Fig. 2 zeigt schematisch ein Reaktionsschema zwischen zwei Lumps im Rahmen der kinetischen Reaktionsmodellierung von Lumps

Fig. 3 zeigt eine schematische Darstellung eines Reaktionsmodells für eine Pyrolyse mit neun Lumps

Fig. 4 zeigt einen Vergleich der kinetischen Zerfallsraten von verschiedenen Kunststoff-Ausgangsmaterialien

Fig. 5A-5I zeigen die Abweichungen der einzelnen Lumps des Reaktionsmodells als Funktion der mittleren Pyrolysetemperatur

Fig. 6 zeigt eine gemessene Siedepunktverteilung (gepunktete Linie) im Vergleich zu einer mittels des Reaktionsmodells berechneten Siedepunktverteilung (durchgehende Linie)

Fig. 7A-7I zeigen eine Parameterstudie zu verschiedenen Pyrolysetemperaturen und Masseflüssen in dem Pyrolyse-Reaktor

**Beispiel** 1

Beispiel 1 betrifft ein sequentielles Reaktionsmodell mit neun Lumps für die Co-Pyrolyse von Kunststoffgemischen mit einer Schwerölfraktion und der Bestimmung der Reaktionsparameter des sequentiellen Reaktionsmodells. Das Kunststoff-Ausgangsmaterial ist in diesem Fall ein das Kunststoffgemisch mit der Schwerölfraktion.

In diesem Beispiel wurde ein kinetisches Reaktionsmodell auf Basis von neun Lumps verwendet. Das Reaktionsmodell weist ausschließlich sequentielle Teilreaktionen ohne alternative Reaktionspfade auf. Dieser Umstand ermöglichte eine unkomplizierte Implementierung des Reaktionsmodells. Das Reaktionsmodell wurde anhand von Versuchsdaten festgelegt, die in einem Pyrolyse-Reaktor in Form eines Rohrreaktors in Laborgröße mit einem maximalen Durchsatz von 2500 g/h gesammelt wurden.

Für die Bestimmung des Reaktionsmodells wurden drei verschiedene Arten von Kunststoffen, gemischt mit einer Schwerölfraktion in unterschiedlicher Zusammensetzung, als Kunststoff-Ausgangsmaterial für den Pyrolyse-Reaktor verwendet. Bei den Kunststoffen handelte es sich um unbehandeltes Polypropylen (PP), Polyethylen niedriger Dichte (LDPE) und Polyethylen hoher Dichte (HDPE) in Pulverform (siehe Tabelle 1). Aufgrund der physikalischen Abmessungen des Reaktors, insbesondere des engen Innendurchmessers der Rohre, musste die maximale Partikelgröße des Kunststoffs kleiner als 500 $\mu$m sein. Aus diesem Grund wurde der Kunststoff vor der Verwendung für die Experimente unter kryogenen Bedingungen gemahlen. Das maximale Verhältnis von Kunststoff zu Trägermedium, das durch die Konstruktion der Anlage erreicht werden konnte, hat 30 Gew.-% betragen. Höhere Kunststoffanteile hätten zu Verstopfungen im Beschickungssystem der Reaktoren geführt.

Das verwendete organische Trägermedium war ein leicht verfügbares Nebenprodukt aus dem Erdölraffinerieprozess. Es handelt sich um ein überwiegend aliphatisches Medium mit einem Aromatengehalt von etwa 25 %, einer Dichte von 880 kg/m$^3$ und einem Heizwert von 45 MJ/kg. Da das Trägermedium unter den im Reaktor herrschenden Bedingungen ebenfalls gecrackt wird, wurden in Vorversuchen die (kinetischen) Reaktionsparameter nur für das Trägermedium ermittelt.

Tabelle 1. Spezifikationen der für die Pyrolyseversuche verwendeten Kunststoffarten (d.h. Kunststoff-Ausgangsprodukten)

| | Molekülmasse (Mw) | Heizwert | Brennwert | TGA Wendepunkt |
|---|---|---|---|---|
| | (g/mol) | (kJ/kg) | (kJ/kg) | (°C) |
| Polypropylen | $3.624 \cdot 10^5$ | 44.510 | 47.343 | 484 |
| LD-Polyethylen | $2.3834 \cdot 10^5$ | 43.409 | 46.159 | 500 |
| HD-Polyethylen | $2.0275 \cdot 10^5$ | 43.525 | 46.409 | 509 |

Versuchsaufbau und Versuchsablauf

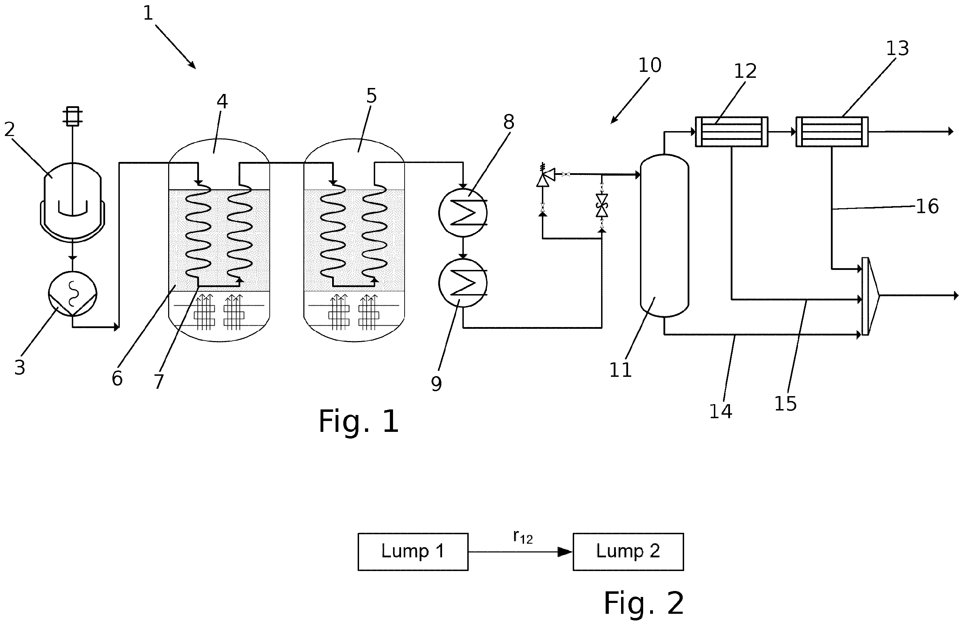

Die Versuche wurden in einem Pyrolyse-Reaktor 1 (auch als Labor-Reaktor bezeichnet) durchgeführt, der speziell für diesen Prozess gebaut wurde und in Fig. 1 schematisch dargestellt ist. Der Pyrolyse-Reaktor 1 entspricht im Wesentlichen dem in Schubert T, Lehner M, Hofer W (2018) Experimental and modeling approach of LDPE thermal cracking for feedstock recycling, 14th MINISYMPOSIUM CHEMICAL & PROCESS ENGINEERING and 5th PARTICLE FORUM Book of Abstracts beschriebenen Reaktor mit einigen Anpassungen des Versuchsablaufs.

Das flüssige Trägermedium und das Kunststoffpulver wurden vor den Versuchsläufen manuell in vordefinierten Massenverhältnissen zwischen 0 und 30 Gew.-% Kunststoff gemischt. Das Gemisch (d.h. das Kunststoff-Ausgangsmaterial) wurde dann in eine Vorratsbehälter 2 gefüllt und dort kontinuierlich gerührt. Das Kunststoff-Ausgangsmaterial wird von dem Vorratsbehälter 2 mittels einer Pumpe 3 (in diesem Fall eine Exzenterschneckenpumpe) gefördert. Im Anschluss an die Pumpe 3 waren zwei Reaktoren 4 und 5 angeordnet. Bei den Reaktoren 4, 5 handelte es sich um Rohrschlangen 6, die in Sandbädern 7 auf den erforderlichen Temperaturbereich von 400 °C bis 550 °C erhitzt wurden. Die Länge jeder Reaktorschlange 6 konnte zwischen 8 m und 24 m variiert werden, um die Verweildauer (d.h. die Pyrolysedauer) ohne Änderung des Strömungsbildes weiter anzupassen. Die eigentliche Pyrolyse fand in den Reaktoren 4 und 5 statt. Nachdem das Medium (d.h. die Kunststoff-Produkte nach der Pyrolyse) die Reaktoren 4,5 passiert hatte, wurde es durch einen Luftkühler 8 und einen Ölkühler 9 auf etwa 90 °C abgekühlt. Der Druck im System konnte mit einem Ventil 10 (entweder mit einem Handventil oder mit einem automatischen Membranventil) reguliert werden, um die Produkte auf Atmosphärendruck zu entspannen. Für alle Versuche wurde der Druck im System auf 15 bar geregelt. Im Anschluss an den Ölkühler 9 und die Ventile 10, war eine Flash-Vessel 11 (dt. Verdampfer) angeordnet. Die Kunststoff-Produkte, die bei den Bedingungen in der Flash Vessel 11 gasförmig waren, haben dieses durch ein Kopfteil verlassen und wurden zu einer ersten Kühlfalle 12 und einer zweiten Kühlfalle 13 geleitet. Die erste Kühlfallen 12 war auf eine Temperatur von 15 °C eingestellt, während die zweite Kühlfalle 13 auf eine Temperatur von 0 °C eingestellt war. Kunststoff-Produkte, die am Boden der Flash Vessel 11 vorliegen, werden als Sumpfprodukte bezeichnet und haben die Flash-Vessel durch eine Sumpf-Leitung 14 verlassen bezeichnet. Kunststoff-Produkte, die mittels der ersten Kühlfalle 12 gesammelt wurden, werden als Kopfprodukte bezeichnet und haben die erste Kühlfalle 12 durch eine Kopfleitung 15 verlassen. Kunststoff-Produkte, die mittels der zweiten Kühlfalle 13 gesammelt wurden, werden als Leichtprodukte bezeichnet und haben die zweite Kühlfalle 13 durch eine Leichtprodukte-Leitung 16 verlassen. Die Pyrolyseprodukte (d.h. die Kunststoff-Produkte), die nach der zweiten Kühlfalle 13 unterhalb von 0 °C gasförmig waren, wurden mit einem Gasballon (nicht dargestellt) beprobt. Nach der Produktentnahme wurden alle Flüssigkeiten gewogen, in einem Gefrierschrank gekühlt und zu dem flüssigen Endprodukt gemischt, das analysiert wurde.

Die Versuchsbedingungen, d.h. die Pyrolysetemperatur und die Pyrolysedauer wurden hauptsächlich durch die Temperatur der Sandbäder 6, die Länge der Reaktoren 4, 5 und die Leistung der Pumpe 3 variiert. Die Pumpenleistung regelte den Massenstrom in den Reaktoren 4, 5 und hat einen Mindeststrom von 300 g/h und einen Höchststrom von 2500 g/h. Daraus ergeben sich Verweildauern (d.h. Pyrolysedauern) in einem Bereich von 3 min bis 60 min, je nach Betriebsbedingungen.

$$Verweildauer = \frac{Reaktorvolumen}{Volumenstrom}$$

Die Verweildauer kann differenziell für jedes Reaktorvolumen-Element berechnet und aufsummiert werden.

Ein Haupteinfluss auf die Verweildauer (die der Pyrolysedauer entspricht) ist die Temperatur des Reaktors, da der Dampfanteil des Mediums und damit seine durchschnittliche Dichte stark von ihr abhängt. In Bezug auf die chemischen Reaktionen sind die Verweildauer und die Temperatur unabhängige Parameter für die Reaktionsrate. In einen Strömungsrohr treten aber auch physikalische Phänomene auf, die zu einer Korrelation der beiden Parameter führen können. Durch die steigende Temperaturen könnte beispielsweise der Siedepunkt teilweise überschritten werden, weshalb sich Dämpfe bilden könnten. Zusätzlich könnten auch Reaktionen zu leichteren Produkten (oder auch niedrigsiedenden Lumps) auftreten, wodurch der Dampfanteil steigen würde. Dadurch könnte die Durchschnittliche Dichte sinken. Dadurch würde der Volumentstrom erhöht werden, was bei gleichbleibenden Reaktorvolumen zu einer kürzeren Verweilzeit führen könnte.

Die Analyse des gasförmigen Kunststoff-Produkts, das mittels des Gasballons gesammelt wurde, erfolgte mittels Gaschromatographie nach DIN 51666:2007-01. Die Ergebnisse sind der Heizwert, das spezifische Gewicht und die molekulare Zusammensetzung der Gasphase. Die wahre Siedepunktskurve der flüssigen Produkte und des Trägermediums wurde gemäß ASTM D7169-20e1 mittels simulierter Destillation (SIM-Dist) analysiert.

Reaktionsmodell mit 9 Lumps

Die kinetische Modellierung mittels Lumps ist eine Standardansatz für die Modellierung der Kinetik der Spaltung von Kohlenwasserstoffen. Dieser Ansatz ist notwendig, weil das typische Ausgangsmaterial für die Kohlenwasserstoffpyrolyse ein Gemisch aus vielen verschiedenen Molekülen ist und die Berücksichtigung jeder einzelnen realen Reaktion zwischen jedem Molekül nicht realisierbar ist. Bei diesem Verfahren wird jede einzelne Komponente der Kunststoff-Produkte einem bestimmten Lump in einem Reaktionsnetzwerk zugeordnet. Die Aufteilung der Lumps erfolgte nach dem Siedepunkt. Die Aufteilung kann alternativ auch nach anderen Materialeigenschaften wie Molekularstruktur oder Dichte vorgenommen werden. Jeder Lump fungiert als Pseudo-Komponente mit repräsentativen Materialeigenschaften, die von den darin enthaltenen Molekülen abgeleitet sind. Die Spaltreaktionen zwischen den Lumps wurden als irreversible, einstufige, monomolekulare Reaktionen modelliert, wie in Fig. 2 schematisch dargestellt ist, wobei die Reaktionsgeschwindigkeit r (vgl. Gl. (1)) dem Arrhenius-Gesetz (vgl. Gl. (2)) folgt.

$$r_{12} = k_{12} \cdot X_{Lump1} \qquad (1)$$

$$k_{12} = k_{12}^* \cdot e^{-\frac{E_{A12}}{R \cdot T}} \qquad (2)$$

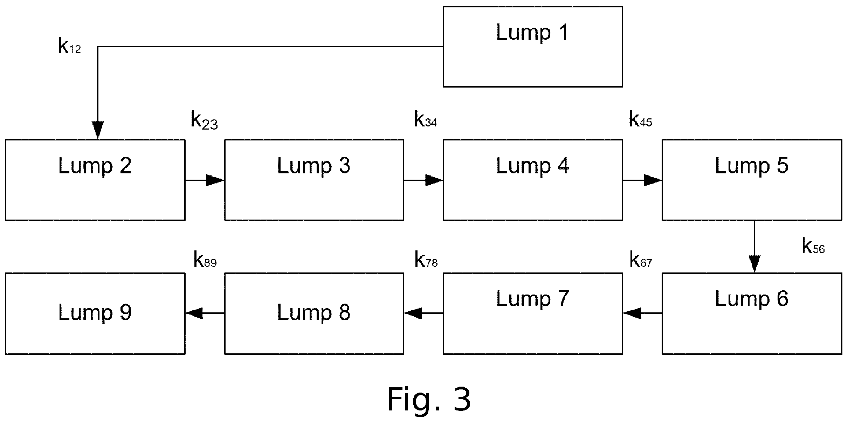

Bei dem Reaktionsmodell in diesem Beispiel wurden die Lumps nach dem Siedepunkt getrennt. Zunächst wurden die für die Raffinerie interessanten Siedepunkt-Temperaturbereiche definiert, was zu der in Tabelle 2 dargestellten Klassifizierung von Gas bis Sumpfprodukte führte. Als Plastik/Wachs-Lumps wurden alle Komponenten mit einem Siedepunkt über 600 °C definiert, was das Ende des Siedepunkt-Temperaturbereichs des organischen Trägermediums darstellt. Ein vollständiges Reaktionsnetzwerk mit neun Lumps (d.h. die Anzahl N an Lumps ist neun), bei dem jeder schwerere Lumps (d.h. jeder der Lumps mit höherem Siedepunkt-Temperaturbereich) mit jedem leichteren Lump (d.h. mit jedem der Lumps mit niedrigerem Siedepunkt-Temperaturbereich reagiert), würde aus N*(N-1)/2 also 36 verschiedenen Reaktionen mit jeweils zwei kinetischen Reaktionsparametern für jede Reaktion bestehen. Das sequenzielle Reaktionsmodell berücksichtigt nur eine Reaktion für jeden schwereren Lump auf den nächst leichteren Lump, der in Bezug auf den Siedepunkt direkt folgt. Dadurch verringert sich die Anzahl der Reaktionen erheblich auf acht unbekannte Reaktionen mit 16 kinetischen Parametern. Das daraus resultierende Reaktionsmodell ist schematisch in Fig. 3 dargestellt.

Das sequentielle Reaktionsmodell berücksichtigt die Zersetzung von Gemischen aus Kunststoffen und Trägermedium derart, dass es keine Wechselwirkung zwischen den Materialien gibt. Für das Trägermedium und jeden Kunststoff im Kunststoff-Ausgangsmaterial kann das Reaktionsmodell unabhängig voneinander gelöst werden. Der endgültige Massenanteil des Lumps j ist die Summe aller Lumps mit gleichem Siedepunkt-Temperaturbereich aus n getrennten Komponenten (Gl. (3)).

Tabelle 2. Siedepunkt-Temperaturbereich und Anzahl an Kohlenstoff-Atomen für die neun Lumps des Reaktionsmodells

| Lump Name | Lump Nr. | Siedepunkt-Temperaturbereich | Anzahl an Kohlenstoffatomen |
|---|---|---|---|
| | | (°C) | (-) |
| Plastik/Wachs (P) | 1 | >600 | >55 |
| Sumpfprodukte (Res) | 2 | 450 - 600 | 30-55 |
| Schweröl (HO) | 3 | 400 - < 450 | 23-30 |
| Spindelöl (SO) | 4 | 350 - < 400 | 18-22 |
| Gasöl (GO) | 5 | 225 - < 350 | 13-17 |
| Kerosin (Kero) | 6 | 165 - < 225 | 10-12 |
| Naphtha | 7 | 20 - < 165 | 5-9 |
| LPG | 8 | -120 - < 20 | 2-4 |
| Gas | 9 | < - 120 | 1 |

$$X_j = \sum_{i=1}^{n} X_{j,i} \quad and \quad \sum_{j=1}^{m} X_j = 1 \tag{3}$$

Simulation und Fitten der Reaktionsparameter

Der Reaktor wurde in PetroSim 7.2 als benutzerdefinierte Operationseinheit in Visual Basic programmiert. Für die Simulation wurde die Laboranlage entsprechend ihrer Geometrie in neun Teile aufgeteilt. Jeder Teil wurde als rohrförmiger Pfropfenströmungsreaktor simuliert und unterscheidet sich durch seine Geometrie, die Umgebungstemperatur und die Rohrisolierung von den anderen Teilen. Zum Beispiel ist der erste Teil ein horizontales Rohr, der zweite Teil ein vertikales Rohr und der dritte eine abwärts fließende Schlange. Alle diese Geometrien haben unterschiedliche Formeln für den Wärmeübergangskoeffizienten und unterschiedliche Umgebungstemperaturen. Die Anfangsbedingungen für die Integration des ersten Reaktorteils sind der gemessene Massenstrom, die gemessene Zulauftemperatur und die Konzentration des Zulaufs in Lumps. Die Anfangsbedingungen des nachfolgenden Teils sind die Lösung der Differentialgleichungen des vorhergehenden Teils.

In diesem Modell wurden die Massenbilanz mit der Reaktion der Lumps, die Energiebilanz für die Temperatur des Fluids, die für die Reaktionsraten erforderlich ist, und die Druckverlustgleichung für ein zweiphasiges Fluid gelöst. Die Differentialgleichungen wurden als eindimensionales Gitter entlang der Länge des Reaktorrohrs diskretisiert (vgl. Gl. (4), (5), (6)). Der Darcy-Reibungskoeffizient für die Druckverlustberechnung wird nach der Korrelation von Beggs und Brill für Zweiphasenströmungen berechnet (vgl. (1996) Standard handbook of petroleum & [and] natural gas engineering. Gulf Publ, Houston, TX).

$$\frac{\Delta X_i}{\Delta z} = \frac{-k_i \cdot X_i + k_{i-1} \cdot X_{i-1}}{v} \tag{4}$$

$$\frac{\Delta T}{\Delta z} = \frac{\sum \Delta H_R - \alpha \cdot \frac{4}{d_r} \cdot (T - T_{Umgebung})}{v \cdot \rho \cdot c_p} \tag{5}$$

$$\frac{\Delta p}{\Delta z} = \frac{\lambda \cdot \rho \cdot v^2}{d_r \cdot 2} \tag{6}$$

Die kinetischen Parameter des Modells wurden mit Matlab und dem Surrogat-Optimierungslöser der Toolbox für globale Optimierung angepasst. Die Surrogat-Optimierung wird im Allgemeinen für die globale Optimierung teurer Kostenfunktionen verwendet, für die keine Ableitungen verfügbar sind. Für die Anpassung wurde PetroSim als COM-Server von Matlab angesprochen, um die Versuchsparameter und Messungen in den Koffer zu schreiben und die Ergebnisse nach der Berechnung zu erhalten. Die Simulationsergebnisse sind die Zusammensetzung der Lumps der simulierten Produkte im Vergleich zu der gemessenen realen Zusammensetzung der Lumps. Das Modell selbst wird als Blackbox vom Surrogatlöser behandelt, der keinen Gradienten für die Anpassung benötigt. Die in dieser Studie verwendete Zielfunktion Obj ist die Summe der quadrierten Fehler zwischen der experimentellen und der berechneten Zusammensetzung der Lumps gemäß Gleichung (7).

$$Obj = \sum_{j=1}^{m} \sum_{i=1}^{n} \left( X_{i,j}^{cal} - X_{i,j}^{exp} \right)^2 \qquad (7)$$

Da davon ausgegangen wird, dass es keine Wechselwirkungen zwischen den Komponenten gibt, können die kinetischen Reaktionsparameter zunächst für das Trägermedium und dann für jeden Kunststoff einzeln angepasst werden, indem Versuche nur mit dem Trägermedium bzw. nur mit dem einzelnen Kunststoff (d.h. einem Kunststoff-Ausgangsmaterial mit nur einem Kunststoff) durchgeführt werden. Die Parameterbereiche und die Anzahl der Versuche für jede Kunststoffzusammensetzung sind in Tabelle 3 aufgeführt. Von jedem Kunststoff-Ausgangsmaterial wurden zwei bis drei Versuche nach dem Zufallsprinzip für die Auswertung der kinetischen Parameter ausgewählt. Diese ausgewählten Versuche und alle Versuche mit gemischten Kunststoffen wurden nur für die Bewertung der Modellparameter verwendet, nicht aber für das Training des Modells.

Tabelle 3: Daten für die Anpassung der kinetischen Reaktionsparameter. Für die Auswertung der Ergebnisse wurden gemischte Kunststoffläufe verwendet

| | Anzahl an Versuchen für | | Temperatur Bereich | Massenfluss Bereich | Reaktorlängenbereich | Plastik-Anteil |
|---|---|---|---|---|---|---|
| | Fitten | Evaluierung | | | | |
| | (-) | (-) | (°C) | (g/h) | (m) | (wt.%) |
| Trägermedium | 21 | 3 | 410-520 | 600-2500 | 32-48 | 0 |
| Polypropylen | 10 | 2 | 440-520 | 600-2500 | 32-48 | 10-30 |
| LD-Polyethylen | 20 | 3 | 450-530 | 600-2500 | 48 | 10-30 |
| HD-Polyethylen | 20 | 3 | 450-540 | 600-2500 | 48 | 10-30 |
| PlastikGemisch | 0 | 32 | 440-530 | 600-2500 | 32-48 | 30 |

Simulation und Fitten der Reaktionsparameter

Die experimentellen Ergebnisse zeigten einen bedeutenden Einfluss der Prozessparameter und der Zusammensetzung des Kunststoff-Ausgangsmaterials auf die Siedepunktverteilung der Kunststoff-Produkte nach den Pyrolyseläufen. Ein Überblick über die experimentellen Prozessdaten ist in Tabelle 4 enthalten.

Tabelle 4. Experimentelle Parameter für die Figuren.

| Nr. | Ausgansmaterial | Plastik | Temperatur Reaktor 4 | Temperatur Reaktor 5 | Massenfluss | Verweildauer |
|---|---|---|---|---|---|---|
| (-) | (-) | (wt.%) | (°C) | (°C) | (g/h) | (min) |
| 257 | Carrier (C) | 0 | 450 | 450 | 1050 | ~17 |
| | C+PP | | | | | |
| | C+LDPE | | | | | |
| | C+HDPE | | | | | |
| 275 | C+MIX | 30 | 460 | 470 | 880 | 19.9 |

Zersetzungskinetik der verschiedenen Kunststoffarten

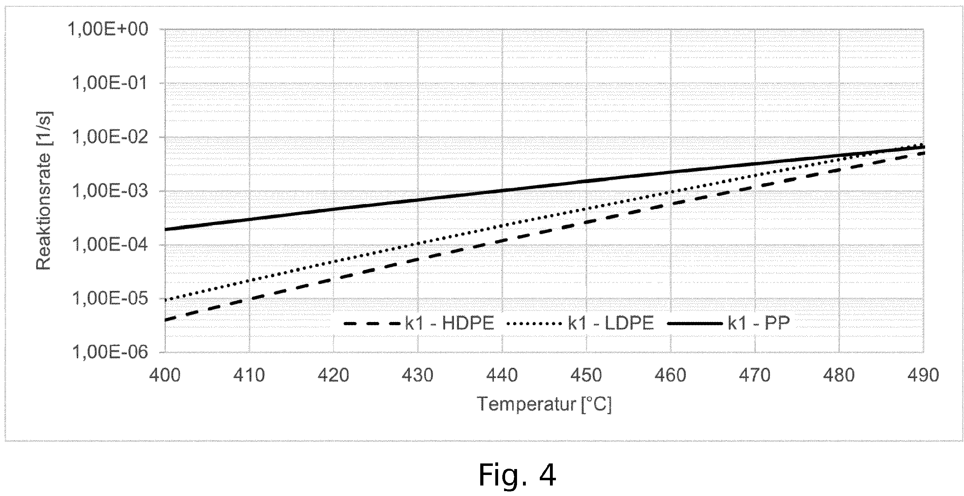

Da der schwerste Lump (d.h. der Lump mit dem höchsten Siedepunkt-Temperaturbereich und den längsten Kohlenwasserstoffen) aus Plastik und dessen schwersten Wachsprodukten besteht, können die k1-Teilreaktionen aus den jeweiligen kinetischen Netzen als die Zersetzungsraten der Kunststoffe angesehen werden. Figur 4 zeigt, dass Polypropylen über den größten Teil des untersuchten Temperaturbereichs viel schneller zerbrochen ist als Polyethylen niedriger Dichte und Polyethylen hoher Dichte. Die Reaktionsgeschwindigkeit von Polyethylen niedriger Dichte ist über den untersuchten Temperaturbereich doppelt so schnell wie die von Polyethylen hoher Dichte. Bei 500 °C nähern sich die Reaktionsgeschwindigkeiten aller Kunststoffe einem ähnlichen Wert und die Unterschiede werden geringer. Die Aktivierungsenergien und Häufigkeitsfaktoren der Reaktionen sind in Tabelle 5 dargestellt.

Tabelle 5. Aktivierungsenergie und Frequenzfaktor der k12-Zersetzungsreaktionen von PP, LDPE und HDPE.

| Reaktion | Frequenzfaktor | Aktivierungsenergie |
|---|---|---|
| | (1/s) | (kj/mol) |
| $k_{12}$ - PP | 1.84E+09 | 167.318 |
| $k_{12}$ - LDPE | 3.21E+19 | 316.124 |
| $k_{12}$ - HDPE | 7.16E+20 | 338.287 |

### Genauigkeit des Reaktionsmodells

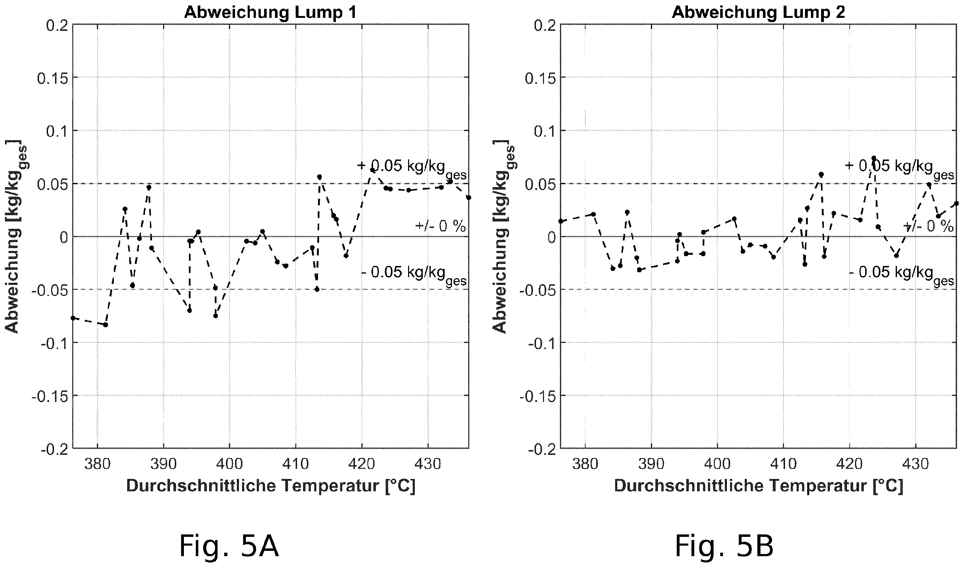

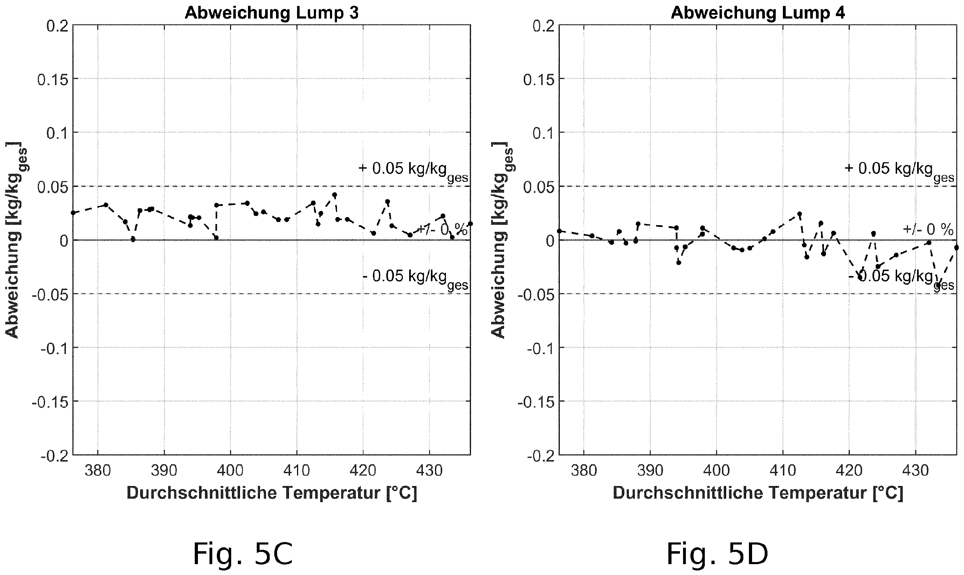

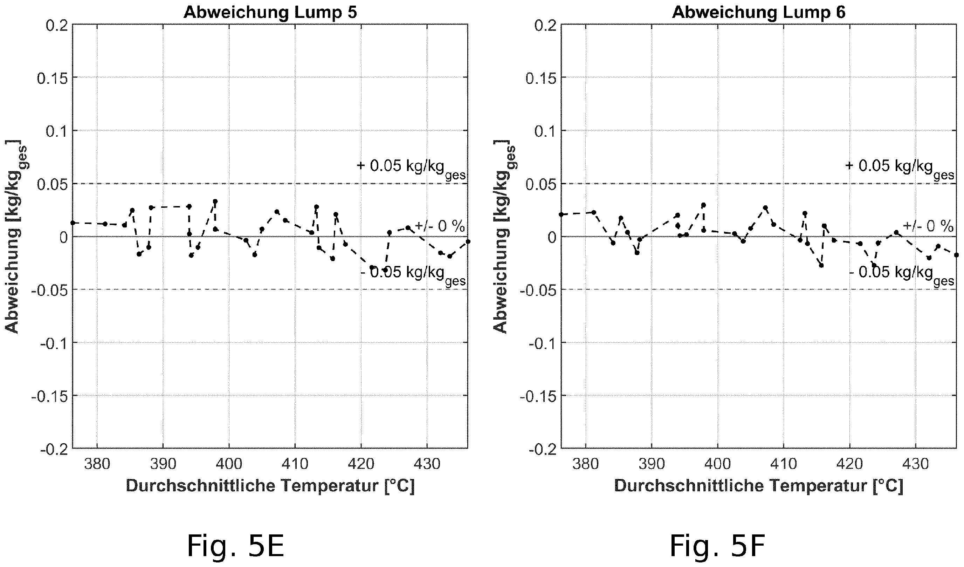

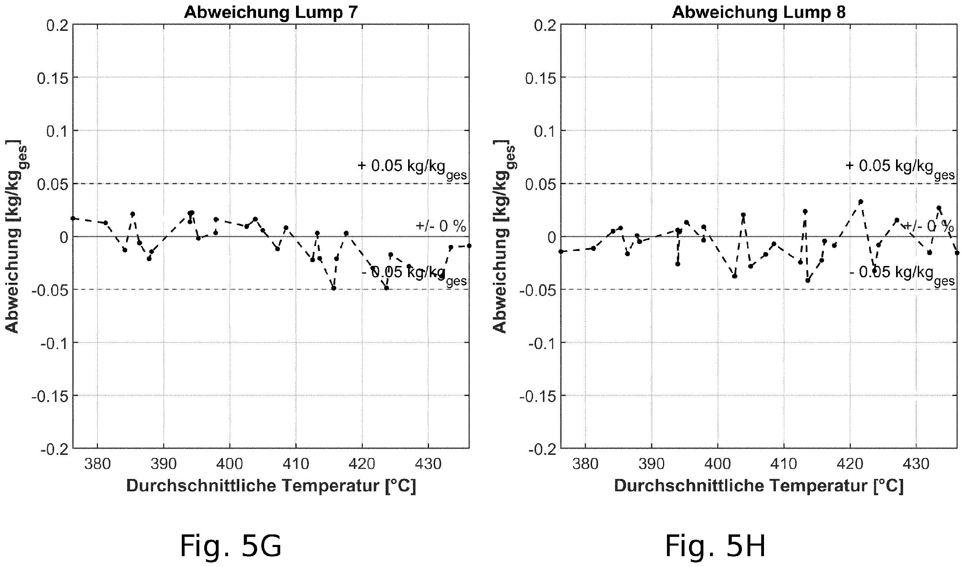

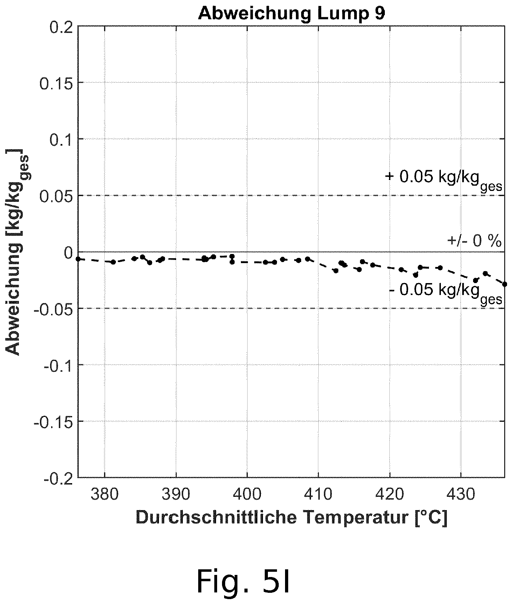

Die berechneten kinetischen Parameter (d.h. die Reaktionsparameter) wurden mit Versuchsdaten bewertet, die nicht für das Fitten der Reaktionsparameter verwendet wurden. Das Kriterium für einen guten Fit des Modells war, dass die maximale Abweichung von den simulierten Daten zu den experimentellen Werten weniger als 0,05 kg/kg beträgt (vgl. Gl. (8)) und kein offensichtlicher systematischer Fehler zu beobachten ist. Die Figuren 5A bis 5I zeigen die Abweichung zwischen dem simulierten und dem gemessenen Massenanteil für jedes Bewertungsexperiment, aufgetragen über die durchschnittliche Temperatur im Reaktor.

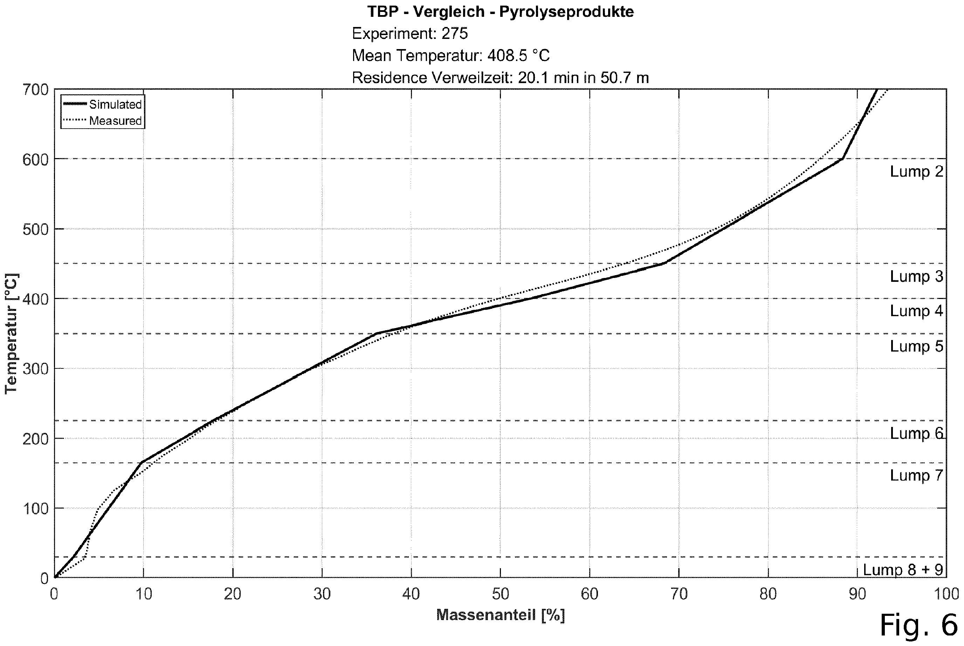

Über alle Lumps und den gesamten Temperaturbereich hinweg ist kein systematischer Fehler zu erkennen. Es ist auch zu erkennen, dass die Genauigkeit bei den meisten Lumps innerhalb des oben definierten Genauigkeitskriteriums liegt. Der am wenigsten genau abgebildete Lump ist der Plastik/Sumpfprodukte-Lump. Bei diesem Lump zeigen zwei der Experimente eine etwas höhere Abweichung als den Schwellenwert mit einer maximalen Abweichung von 0,07 kg/kg. Trotz der höheren Abweichung ist kein systematischer Fehler erkennbar, so dass die kinetischen Parameter trotz dieser beiden Ausreißer gut geeignet sind. Die höhere Abweichung vom schwersten Lump kann mit der Messungenauigkeit von SimDist erklärt werden, die bei höheren Siedepunkten weniger genau ist. Figur 6 zeigt, dass die Siedepunktverteilungen der Simulation (durchgehende Linie) im Vergleich zur gemessenen Siedepunktverteilung (gepunktete Linie) gut übereinstimmen.

$$\Delta X_{Max} = Max\left(\left|X_{j,Simulated} - X_{j,Measured}\right|\right) \leq 0.05 \qquad (8)$$

### Versuchsreihe mit einem Labor-Reaktor

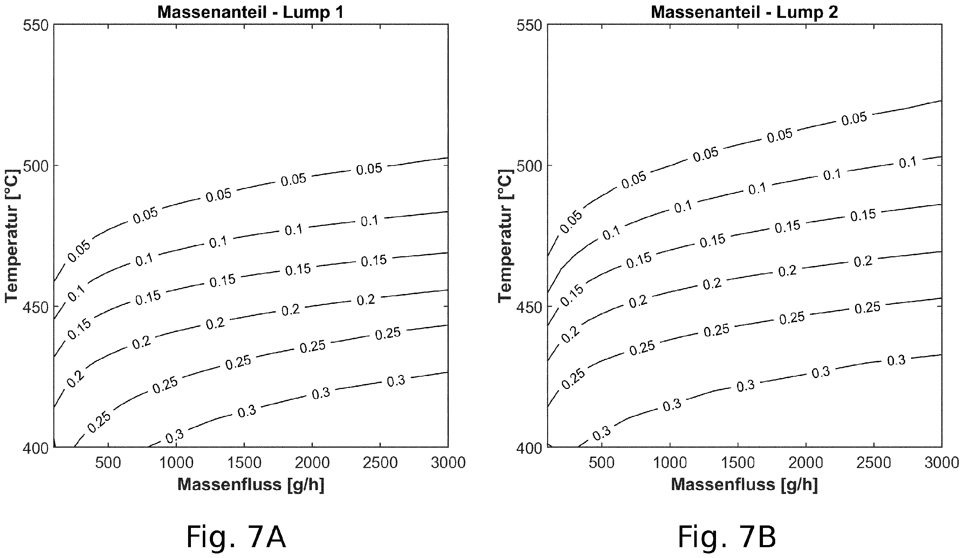

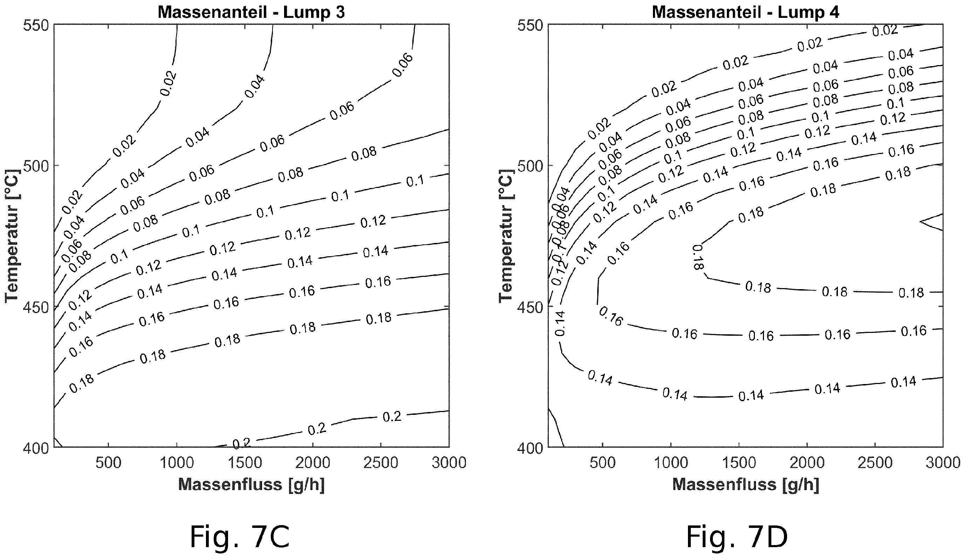

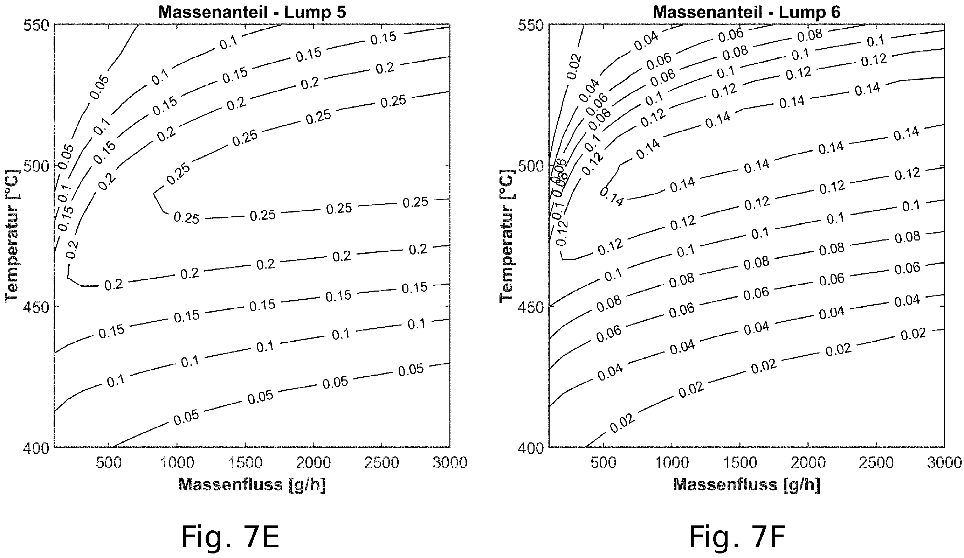

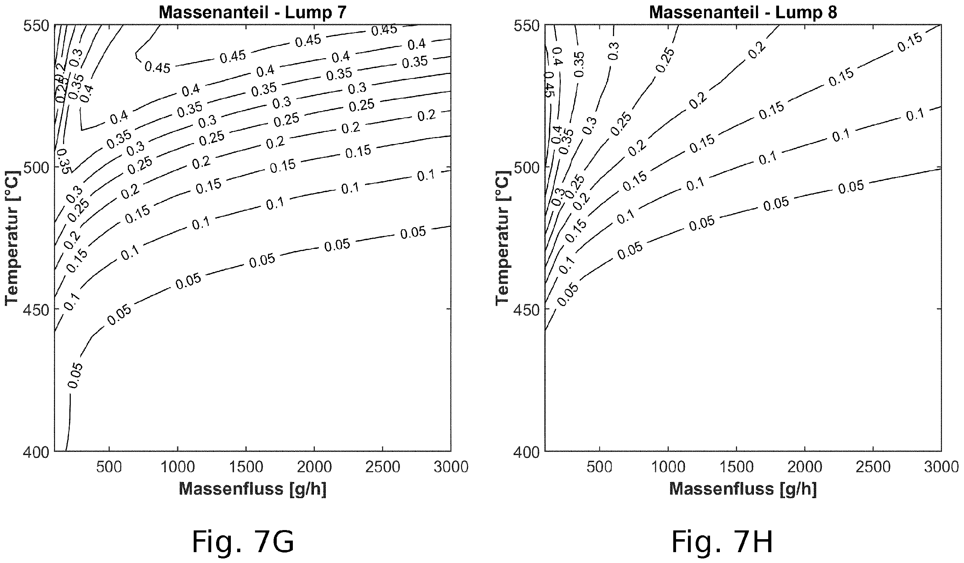

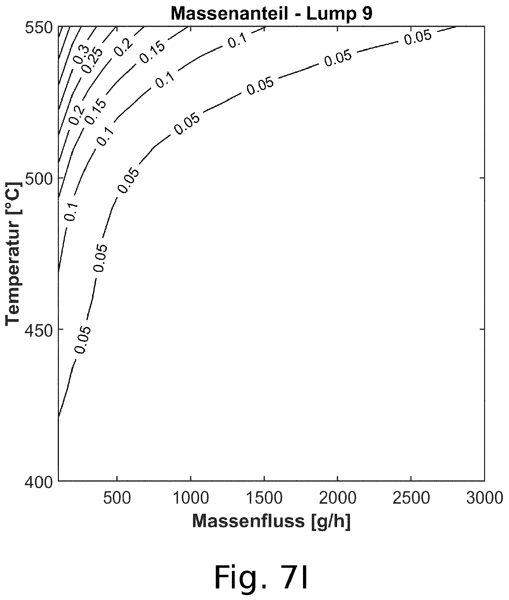

Das kinetische Modell wurde in einer Fallstudie verwendet, um optimale Versuchsparameter für den Labor-Reaktor (der in diesem Fall der Pyrolyse-Reaktor ist) zu finden. Die Fallstudie wurde mit einem Kunststoff-Ausgangsmaterial aus 20 Gew.-% LDPE, 10 Gew.-% PP und 70 % Trägermedium durchgeführt. Für jeden der unterschiedlichen Kunststoff-Ausgangsmaterialien wurde ein separates Reaktionsmodell bestimmt. Die einzelnen Modelle können (entsprechend dem jeweiligen Anteil an Kunststoff-Ausgangsmaterial gewichtet) summiert werden. Die Figuren 7A bis I zeigen die Ausbeute der Lumps des Reaktors über den gesamten Temperatur- und Massenstrombereich. Es ist deutlich zu erkennen, dass Temperaturen über 470 °C einen positiven Einfluss auf die Ausbeute an Kunststoff-Produkten aus wertvolleren Lumps mit Schnittpunkten unter 350 °C haben. Oberhalb dieser Temperatur zerfällt fast der gesamte Kunststoff in die leichteren Fraktionen (bzw. Lumps) Kerosin und Gasöl, die eine deutlich erkennbare Maximalausbeute von 0,14 Gew.-% bzw. 0,25 Gew.-% aufweisen.

### Zusammenfassung des Beispiels 1

Pyrolyseprozesse für das chemische Recycling von Kunststoffen sind eine notwendige Technologie für eine vollständige Kreislaufwirtschaft. In diesem Beispiel wurde gezeigt, dass ein einfaches kinetisches Reaktionsmodell auf Basis von Lumps, das neun Lumps und ausschließlich sequentiellen Teilreaktionen aufweist, die Zersetzung von Polyolefinen in einem Trägermedium mit sehr guter Genauigkeit modellieren kann. Die Auflösung der Siedepunkte der Kunststoff-Produkte ist präziser als bei anderen Reaktionsmodellen mit weniger Lumps, wobei die Anzahl der unbekannten Reaktionsparameter für das Fitten begrenzt ist. Die maximale Abweichung des modellierten Massenanteils von den experimentellen Ergebnissen beträgt für die meisten Lumps weniger als 0,05 kg/kg, mit Ausnahme des Kunststoffreststoff-Lumps, der eine maximale Abweichung von 0,07 kg/kg aufweist. Es wurde nachgewiesen, dass diese Genauigkeit in dem relevanten Temperaturbereich für die langsame Pyrolyse oberhalb von 400 °C und unterhalb von 500 °C nachweisbar ist. Eine Fallstudie für die Laboranlage hat gezeigt, dass es ein deutliches Temperaturfenster zwischen 470 °C und 520 °C für eine maximale Ausbeute an Kerosin oder Gasöl gab.

Abkürzungsverzeichnis

| | |
|---|---|
| C | Trägermedium |
| Cal | Berechneter Wert |
| Exp | Experimenteller gemessener Wert |
| GO | Gasöl |
| HDPE | High-density Polyethylen |
| HO | Schweröl |
| HTC | Wärmeübergangskoeffizient |
| IBP | Initialer Siedepunkt |
| Kero. | Kerosin |
| LDPE | Low-density Polyethylen |
| LKM | Reaktionsmodell mit Lumps |
| LPG | Flüssiggas (Liquified Petroleum Gas) |
| Obj | Zielfunktion |
| P | Plastik |
| PP | Polypropylen |
| Res | Sumpfprodukte |
| SO | Spindelöl |
| TBP | Tatächlicher Siedepunkt |

Symbolverzeichnis

| | |
|---|---|
| C | Anzahl an Kohlenstoffatomen |
| $c_p$ | Wärmekapazität |
| $d_r$ | Durchmesser des Reaktors |
| $Ea_{ij}$ | Aktivierungsenergie für die Umwandlung von Lump i zu Lump j |
| $k^*_{ij}$ | Arrhenius-Konstante (Frequenzfaktor) für die Teilreaktion von Lump i zu Lump j |
| $k_{ij}$ | Geschwindigkeitskonstante für die Teilreaktion von Lump i zu Lump j |
| p | Druck |
| R | Gaskonstante (J/molK) |
| $r_{ij}$ | Teilreaktion von Lump i zu Lump j |
| T | Temperatur |
| v | Flussgeschwindigkeit |
| $X_i$ | Massenanteil von Lump j |
| $X_{ij}$ | Massenanteil vom Lump j der Komponente i |
| z | Länge des Reaktors |
| $\alpha$ | Wärmeübergangskoeffizient |
| $\Delta H_R$ | Reaktionsenthalpie |
| $\lambda$ | Darcy Reibungsfaktor |

(fortgesetzt)

| ρ | Dichte |
|---|---|

**Patentansprüche**

1. Computerimplementiertes Verfahren zur Bestimmung von Werten von Reaktionsparametern eines Reaktionsmodells für eine Pyrolyse eines Kunststoff-Ausgangsmaterials in Kunststoff-Produkte, das Verfahren umfassend die folgenden Schritte:

   - Bereitstellen von Versuchsdaten umfassend eine Mehrzahl von Siedepunktverteilungen von Kunststoff-Produkten, wobei jede Siedepunktverteilung einer Pyrolysetemperatur und einer Pyrolysedauer zugeordnet ist, wobei die Siedepunktverteilungen jeweils in eine Anzahl N Lumps $L_1$ bis $L_N$ gebinnt sind, wobei jedem Lump ein Siedepunkt-Temperaturbereich zugeordnet ist;
   - Bestimmen jeweils eines Wertes der Reaktionsparameter durch Fitten der Reaktionsparameter des Reaktionsmodells an die Versuchsdaten;

   wobei das Reaktionsmodell für jeden Lump $L_i$ von $L_1$ bis $L_{N-1}$ eine durch mindestens einen Reaktionsparameter bestimmte Teilreaktion zur Beschreibung einer Umwandlung von Kunststoff-Produkten dieses Lumps in Kunststoff-Produkte eines in Bezug auf den Siedepunkt-Temperaturbereich darunter liegenden und angrenzenden Lump $L_{i+1}$ aufweist, wobei das Reaktionsmodell weniger als N*(N-1)/2 Teilreaktionen aufweist.

2. Computerimplementiertes Verfahren nach Anspruch 1, **dadurch gekennzeichnet, dass** die Anzahl N an Lumps zumindest sieben beträgt.

3. Computerimplementiertes Verfahren nach einem der Ansprüche 1 oder 2, **dadurch gekennzeichnet, dass** das Reaktionsmodel genau (N-1) Teilreaktionen aufweist.

4. Verfahren zur Bestimmung von Werten von Reaktionsparametern eines Reaktionsmodells für eine Pyrolyse eines Kunststoff-Ausgangsmaterials in Kunststoff-Produkte mit einem computerimplementierten Verfahren nach einem der vorhergehenden Ansprüche, **dadurch gekennzeichnet, dass** das Bereitstellen von Versuchsdaten folgende Schritte umfasst:

   - Durchführen einer Mehrzahl von Pyrolyseläufen mit unterschiedlicher Pyrolysetemperatur und/oder Pyrolysedauer in einem Pyrolyse-Reaktor
   - Messung einer Siedepunktverteilung von Kunststoff-Produkten pro Pyrolyselauf, wobei jede Siedepunktverteilung einer Pyrolysetemperatur und einer Pyrolysedauer zugeordnet ist;
   - Binning der einzelnen Siedepunktverteilungen in die N Lumps $L_1$ bis $L_N$, wobei jedem Lump ein Siedepunkt-Temperaturbereich zugeordnet ist, um Versuchsdaten zu erhalten.

5. Verfahren zur Durchführung einer Pyrolyse eines Kunststoff-Ausgangsmaterials in Kunststoff-Produkte umfassend die folgenden Schritte:

   - Bestimmung von Werten von Reaktionsparametern eines Reaktionsmodells der Pyrolyse durch das computerimplementierte Verfahren gemäß einem der Ansprüche 1 bis 3 oder das Verfahren nach Anspruch 4;
   - Vorgeben einer vorgesehenen Siedepunktverteilung;
   - Festlegen einer Pyrolysetemperatur und einer Pyrolysedauer durch Minimieren eines Abstands einer mittels des Reaktionsmodells und der zuvor bestimmten Werte der Reaktionsparameter berechneten Siedepunktverteilung zu der vorgesehenen Siedepunktverteilung; und
   - Durchführen der Pyrolyse mit der festgelegten Pyrolysetemperatur und der festgelegten Pyrolysedauer.

6. System zur Datenverarbeitung, **gekennzeichnet durch** Mittel zur Ausführung der Schritte des computerimplementierten Verfahrens nach einem der Ansprüche 1 bis 3.

7. System zur Datenverarbeitung nach Anspruch 6, **dadurch gekennzeichnet, dass** die Mittel weiters zur Ausführung des folgenden Schritts eingerichtet sind:

- Berechnen einer Pyrolysetemperatur und einer Pyrolysedauer mittels des Reaktionsmodells und der zuvor bestimmten Werte der Reaktionsparameter, wobei die Pyrolysetemperatur und die Pyrolysedauer derart festgelegt ist, dass ein Abstand einer mittels des Reaktionsmodells berechneten Siedepunktverteilung zu einer vorgesehenen Siedepunktverteilung minimiert ist.

8. Computerprogrammprodukt, umfassend Befehle, die bei der Ausführung des Programms durch einen Computer diesen veranlassen, die Schritte des computerimplementierten Verfahrens nach einem der Ansprüche 1 bis 3 auszuführen.

Fig. 1

Fig. 2

EP 4 462 440 A1

Fig. 3

Fig. 4

Fig. 5B

Fig. 5A

Fig. 5C

Fig. 5D

EP 4 462 440 A1

Fig. 5E

Fig. 5F

EP 4 462 440 A1

Fig. 5G

Fig. 5H

**Abweichung Lump 9**

Fig. 5I

**TBP - Vergleich - Pyrolyseprodukte**
Experiment: 275
Mean Temperatur: 408.5 °C
Residence Verweilzeit: 20.1 min in 50.7 m

Fig. 6

Fig. 7A

Fig. 7B

**Massenanteil - Lump 3**

Fig. 7C

**Massenanteil - Lump 4**

Fig. 7D

Fig. 7F

Fig. 7E

28

Fig. 7H

Fig. 7G

Fig. 7I

Europäisches
Patentamt

European
Patent Office

Office européen
des brevets

# EUROPÄISCHER RECHERCHENBERICHT

**Nummer der Anmeldung**

**EP 23 17 3133**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | SCHUBERT TERESA ET AL: "4-Lump kinetic model of the co-pyrolysis of LDPE and a heavy petroleum fraction", FUEL, IPC SIENCE AND TECHNOLOGY PRESS , GUILDFORD, GB, Bd. 262, 13. November 2019 (2019-11-13), XP085933912, ISSN: 0016-2361, DOI: 10.1016/J.FUEL.2019.116597 [gefunden am 2019-11-13] * Zusammenfassung; Abbildungen 2, 7, 8 * * Seite 3, Spalte 1, Absatz 3 - Spalte 2, Absatz 2; Tabellen 1,2 * * Seite 3, Zeilen 19-21 * ----- | 1-8 | INV. G16C20/10 |
| X,D | LECHLEITNER ANDREAS E. ET AL: "Lumped Kinetic Modeling of Polypropylene and Polyethylene Co-Pyrolysis in Tubular Reactors", PROCESSES, Bd. 9, Nr. 1, 25. Dezember 2020 (2020-12-25), Seite 34, XP093092487, DOI: 10.3390/pr9010034 * Zusammenfassung; Abbildung 2 * ----- | 1-8 | RECHERCHIERTE SACHGEBIETE (IPC) G16C |

Der vorliegende Recherchenbericht wurde für alle Patentansprüche erstellt

| Recherchenort | Abschlußdatum der Recherche | Prüfer |
|---|---|---|
| Den Haag | 17. Oktober 2023 | Nurmi, Jussi |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument
......................................................................................
& : Mitglied der gleichen Patentfamilie, übereinstimmendes
  Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LECHLEITNER, A.E. ; SCHUBERT, T. ; HOFER, W. ; LEHNER, M.** Lumped Kinetic Modeling of Polypropylene and Polyethylene Co-Pyrolysis in Tubular Reactors. *Processes,* 2021, vol. 9, 34, https://doi.org/10.3390/pr9010034
- **SCHUBERT, TERESA et al.** 4-Lump kinetic model of the co-pyrolysis of LDPE and a heavy petroleum fraction. *Fuel,* 2020, vol. 262, 116597
- **DESAVATH V. et al.** Kinetic modeling for catalytic cracking of pyrolysis oils with VGO in a FCC unit. *Chemical Engineering Science,* 2017, vol. 170, 790-798
- **LECHLEITNER et al.** *Processes,* 2021, vol. 9, 34
- Experimental and modeling approach of LDPE thermal cracking for feedstock recycling. **SCHUBERT T ; LEHNER M ; HOFER W.** 14th MINISYMPOSIUM CHEMICAL & PROCESS ENGINEERING and 5th PARTICLE FORUM Book of Abstracts. 2018